Europäisches Patentamt

European Patent Office

Office européen des brevets

(19)

(11) **EP 1 090 634 A1**

(12) **EUROPEAN PATENT APPLICATION**

(43) Date of publication:
**11.04.2001 Bulletin 2001/15**

(51) Int Cl.⁷: **A61K 31/14**, A61K 38/08,
A61P 3/04, A61P 3/06

(21) Application number: **99810892.2**

(22) Date of filing: **01.10.1999**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI**<br><br>(71) Applicant: **Hauser, Helmut**<br>**8092 Zürich (CH)**<br><br>(72) Inventors:<br>• **Hauser, Helmut**<br>**8713 Uerikon (CH)** | • **Werder, Moritz**<br>**8918 Unterlunkhofen (CH)**<br><br>(74) Representative: **Blum, Rudolf Emil Ernst**<br>**c/o E. Blum & Co**<br>**Patentanwälte**<br>**Vorderberg 11**<br>**8044 Zürich (CH)** |

(54) **Agents for reducing cholesterol and lipid uptake**

(57) An active substance is described that is suitable for the reduction of cholesterol and lipid uptake from the gut. In order to act as precipitant, said agent is characterized by the presence of a cation having at least two positive charges thus positioned to allow precipitation of cholesterol and lipids comprising bile salt micelles and unilamellar vesicles in the gut.

EP 1 090 634 A1

Printed by Jouve, 75001 PARIS (FR)

**Description**

This invention relates to agents for the use in medicine, in particular for reducing uptake of cholesterol and other dietary lipids from the gut. The agents of the present invention therefore have application in hyperlipidaemia, including hypercholesterolaemia, and the management of obesity.

Cholesterol, on the one hand, is an essential constituent of the plasma membrane of cells, on the other hand, in the case of high levels of blood cholesterol, deposition thereof in the wall of arteries can occur, favoring atherosclerotic plaque, myocardial infarction and stroke. In view of the high number of deaths caused by atherosclerosis in western industrialized nations, there is a great need for agents reducing the risk of such diseases.

A large amount of cholesterol, about 1/3, in the body is of dietary origin taken up from the gut. Since cholesterol and other dietary lipids are insoluble in water and aqueous media they have to be transported therein dispersed in a stable form. Said stably dispersed form are either bile salt micelles or small unilamellar phospholipid vesicles. These lipid particles function as donors or carriers of water-insoluble dietary lipids in the lumen of the small intestine. Hitherto made efforts of reducing or inhibiting cholesterol absorption in the gut and in turn lowering cholesterol blood levels comprise e.g. the administration of saponins as dietary supplement. (Harwood et al., Journal of Lipid Research 34, 377-395 (1993)). This method, however, can scarcely be applied due to problems to obtain pure saponins in sufficient quantities from natural sources.

It is also already known that the absorption of cholesterol, esters of cholesterol and other dietary lipids by the brush border membrane ("BBM") of epithelial cells in the gut is protein-mediated (Turnhofer and Hauser, Biochemistry 29, 2142-2148 (1990); Compassi et al., Biochemistry 34, 16473-16482 (1995); Turnhofer et al., Biochim. Biophys. Acta. 1024, 249-262 (1990); Hauser et al., Biochemistry 37, 17843-17850 (1998)). From W097/36927 it is known that amphipathic molecules interact with the proteins involved in lipid absorption and thus inhibit lipid absorption (Boffelli et al., FEBS Letters 411, 7-11 (1997)).

However, there is still a need to improve the reduction in cholesterol and lipid uptake.

Hence, it is a general object of the invention to provide active substances and compositions comprising said substances that allow reduction of the cholesterol and lipid uptake from the gut.

It has now surprisingly be found that the cholesterol and lipid carrying bile salt micelles (further on referred to as micelles) and small unilamellar phospholipid vesicles (further on referred to as vesicles) can be precipitated by agents providing at least two positive charges.

Thus, the precipitant of the present invention that allows the reduction of cholesterol and lipid uptake is characterized by the presence of at least two charges in the same cation.

The at least two charges can be present in the precipitant to be administered to a patient, or a respective administration form, e.g. a tablet, can comprise an uncharged precursor of an at least doubly charged cation. Conversion of such an uncharged precursor to the cation can e.g. occure in the stomach.

Figure 1A shows a β-peptide with precipitation effect only (TFA = trifluoroacetic acid).

Figure 1B shows a β-peptide with precipitation and inhibitor effect (TFA = trifluoroacetic acid).

Figure 1C shows a β-peptide with precipitation and inhibitor effect (TFA = trifluoroacetic acid).

Figure 1D shows a β-peptide with inhibitor effect only (TFA = trifluoroacetic acid).

Figure 2 shows the efficiency of a precipitant of the present invention, namely polylysine with an average molecular weight of 21600; in said Figure, Cholesterol in Sup (%) means the percentage cholesterol remaining in the supernatant after centrifugation; A (500 nm) means absorption at 500 nm; Poly K (mM) means the amount of polylysine added; the arrows indicates the theoretical concentration of precipitant at which charge neutralization occurs.

Figure 3 shows the efficiency of a precipitant of the present invention, namely polylysine with an average molecular weight of 2300; in said Figure, Cholesterol in Sup (%) means the percentage cholesterol remaining in the supernatant after centrifugation; A (500 nm) means absorption at 500 nm; Poly K (mM) means the amount of polylysine added; the arrows indicates the theoretical concentration of precipitant at which charge neutralization occurs.

It could be shown, that by the application of the precipitant of the present invention to cholesterol and lipid carrying particles having a negatively charged surface, said particles can be precipitated.

It could be shown by in vitro tests that the amount of ionic agent needed to get quantitative precipitation of micelles is dependent not only on the charges carried by one molecule but also on the design of a specific cation implying that several factors are involved in such a precipitation reaction. On the one hand, there is of course an electrostatic interaction between the negatively charged micelles and vesicles and the cations. On the other hand, a second aspect involved is likely to be a "chelate effect", and a third aspect is a "linking" of two or more micelles/vesicles by the at least doubly charged cation.

"Chelating effect" in the scope of the present text, means that by binding one of the charged groups one or more further charged groups of the same molecule are brought in close contact to the micellar surface whereby their binding is favoured.

Suitable cations for the purposes of the present invention therefore are metal ions such as $Ca^{2+}$, but also

$Ni^{2+}$, $Fe^{2+}$ and $Fe^{3+}$, possibly in the form of charged coordination complexes thereof having a geometry that allows the desired particle linkage.

Further suitable and preferred cations are in particular quaternary ammonium groups carrying compounds. Such compounds are preferably protonated polyamino compounds, e.g. diprotonated dodecane diamine, or at least partially protonated alkane polyamines, such as respectively substituted polyalkyls, but also respectively substituted other oligomers and polymers, whereby the length of the chain, the distance between the quaternary ammonium groups and possibly further substituents can be varied and largely influences the efficiency of the precipitant. It has e.g. be found that for diamines, a chain length of 12 carbon atoms is suitable, while a chain length of 6 or 10 proved to be unsatisfactory in the performed in vitro test. However, in any case, for many types of molecules, cations with more than 2 suitably placed positively charged groups proved to be advantageous, since beside of the possibility to simultaneously profit from the chelate and the linking effect, the water solubility is enhanced.

Another group of possible precipitants are suitably substituted proteins and peptides, such as positively charged proteins and peptides, as well as positively charged β-peptides (oligomers of β-amino acids) capable of forming helical structures (usually at least about 6 residues are needed, (for further information see e.g. Guichard et al., Helv. Chim. Acta 81, 187 (1998); Abele et al. ibid. 81, 2141 (1998); Seebach et al. Angew. Chem. 111, 1700-1703 (1999); ibid., Int. Ed. 38, 1595 (1999)) and having amphipathic character. Such proteins and peptides may also comprise synthetic amino acids.

It is already known that certain amphipathic proteins and peptides (with a hydrophobic and a hydrophilic side) can inhibit proteins involved in cholesterol and lipid resorption (WO 97/36927). Amphipathic and non-amphipathic proteins and peptides with regions comprising positive charges are also suitable to act as precipitant for cholesterol and lipids comprising micelles and vesicles. Relevant for their activity is the presence of at least two actual positive charges (the charge separation in amino acids does not - or at least not sufficiently - contribute to the precipitating effect) in suitable distance.

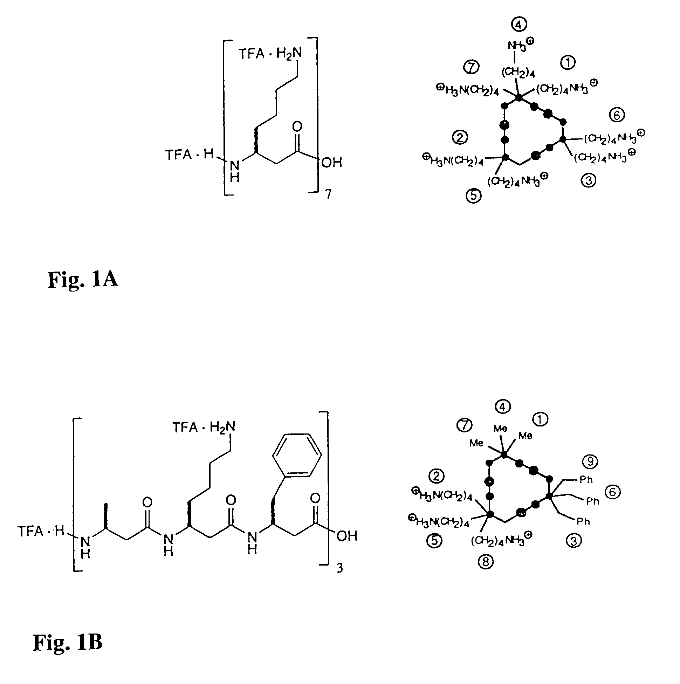

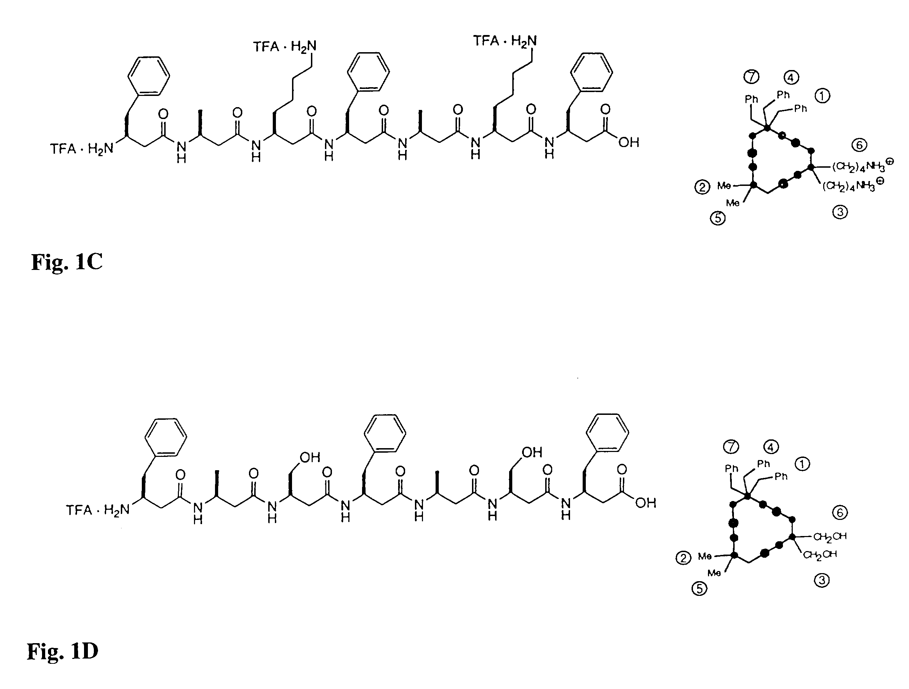

It has e.g. be found that β-peptides capable of forming helical structures and carrying at least two positive charges in suitable positions are suitable precipitants. Such β-peptides may also have amphipathic character. For β-peptides forming helices and having amphipathic character, be it charged or uncharged β-peptides, it has also been found that they are good inhibitors of the promoting protein (see Hauser et al., Biochemistry 37, 17843-17850 (1998) for description of the protein). Thus, β-peptides can be designed acting primarily as precipitant (see e.g. Figure 1A for an example for a β-peptide with helical, non-amphipathic structure), as inhibitor (see e.g. Figure 1D for an example for a β-peptide with helical, amphipathic structure but without positive charges) or as a combination of both types (see e. g. Figures 1B and 1C for examples of β-peptides with helical, amphipathic structures comprising charges), a possibility that also exists for amphipathic proteins and peptides.

A great advantage of β-peptides is that they are resistant to all kinds of mammalian proteases and peptidases, including the most aggressive ones such as pronase and proteinase K (T. Hintermann, D. Seebach, Chimia 51, 244-247 (1997)).

Another type of peptides or proteins suitable as precipitants are chains built of natural and/or synthetic positively charged amino acids. Naturally occurring charged amino acids are lysine and argenine, that can be present in mixed or homo chains, such as poly lysine that is commercially available in several average chain lengths.

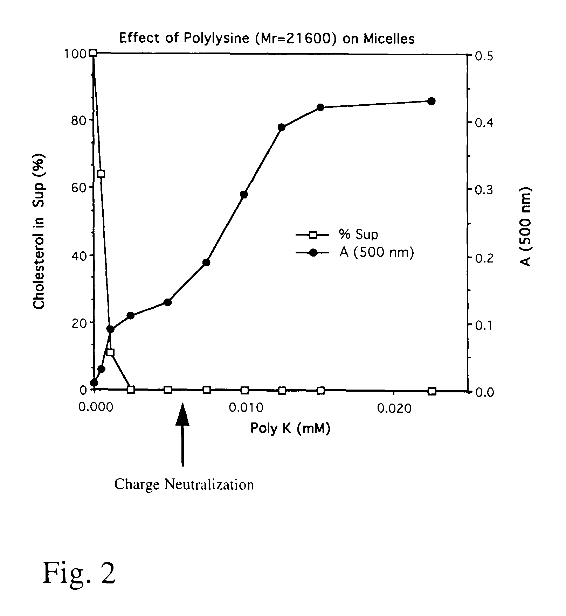

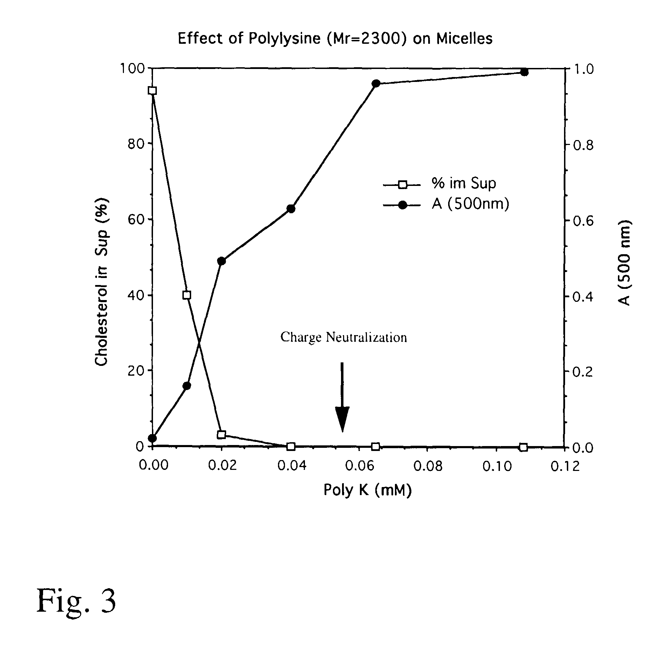

Tests performed with e.g. polylysine showed that a full precipitation is obtained at about the point of charge equivalence. In order to determine the efficiency of specific cations, an in vitro test was used in which micelles comprising radio labeled cholesterol (cholesterol and micelles were produced according to known methods, see below) were generated. To a predetermined amount of such micelles, the respective precipitant was added in portions, and the precipitation formed upon addition of specific amounts of precipitant measured by two methods, namely by light scattering and by determination of the radioactivity in the supernatant after centrifugation. This test, e.g. for polylysines with an average molecular weight of 2300 and 21'600 showed 100 % precipitation at the amount calculated for charge equivalence (see Figure 3, equivalence point: poly K = 0.056 mM; Figure 2, equivalence point: poly K = 0.0061 mM) whereby the actually used amount is not critical, since almost full precipitation already occures with a smaller amount, and since the precipitate remains precipitated in the presence of excess precipitant. The light scattering showed an increase over the point of charge equivalence, indicating that - although all micelles are precipitated by at least the equivalence amount, and remain precipitated - the kind of the precipitation is still altered.

The fact that for a great variety of precipitants the precipitation is not dependent on an exactly predetermined amount of precipitant is of great importance for drug design and administration.

Another very important aspect of the present invention is that the precipitant can be designed to provide the precipitate with specific characteristics, e.g. by using specific amino acids for building up β-peptides providing the micelle distant "surface" of the precipitate with specific features facilitating the cholesterol and lipid excretion.

The counter anion(s) - if present - are not critical, however, pharmaceutically acceptable hydrated strong acids are preferred such as strong organic acids, chloride, phosphates, etc.

A preferred method of administration of the precipitants of the present invention is oral administration. Although the wide variety of possible precipitants comprises cations that are not or not fully digested in the intestine, also digestible/degradable agents can be used, provided that they are administered in a composition designed for drug release in the gut. It may furthermore be desired for specific applications to administer the agent of the present invention in a form intended for slow release or continuous release. Such galenic formulations are known.

The present invention is now further described by means of examples.

Examples

**General Procedure**

A micellar dispersion consisting of 5 mM taurocholate, 0.6 mM oleic acid and 0.1 mM radiolabeled cholesterol was prepared as described before (Schulthess et al., J. Lipid Res. 37, 2405-19 (1996)) using Mg- and Ca-free phosphate-buffered saline (PBS) from Life Technologies, Basel, Switzerland. This stock solution was diluted by adding various amounts of precipitant in PBS, and PBS was added to a final volume of 1 ml. The mixture was shortly vortexed and incubated at room temperature for about 5 min before measuring its optical density (turbidity) at 500 nm. Precipitation (in %) was determined as follows: an aliquot of 200 μl of the incubation mixture was centrifuged for 3 min. at 100'000 g and the radioactivity of 100 μl supernatant was determined in a liquid scintillation counter. The radioactivity of 100 μl of the original mixture was also determined without centrifugation. The precipitation was determined as

$$\text{precipitation (\%)} = 100 \, [1-(X/A)]$$

A = radioactivity in supernatant without centrifugation
B = radioactivity in supernatant after centrifugation

As a control: the precipitation (%) is determined in the absence of precipitant and a value of precipitation (%) = $2 \pm 1$ is obtained.

The performed experiments and the results are further described in Table 1.

Table 1 shows, that while cations with a single positive charge do not result in a precipitation, they may negatively influence "week" precipitants such as doubly charged metal cations (see Ca$^2$ + Tris, a mono-amino compound). The results furthermore show the advantage of compounds carrying more than two quaternary ammonium groups over those with only one such group.

Table 1

| Substance | Charge ratio (Taurocholate/ precipitant) | Precipitation (%) | Turbidity (OD 500 nm) |
|---|---|---|---|
|  |  |  |  |
| β-peptide 1 * | $1 \pm 0.5$ | 100 | not determined |
| β-peptide 2 ** | $1.1 \pm 0.1$ | 100 | $1 \pm 0.1$ (max) |
| β-peptide 3*** | $1.4 \pm 0.5$ | 100 | not determined |
|  |  |  |  |
| Polylysine Mr = 2300 | $1.4 \pm 0.6$ | 100 | $0.6 \pm 0.2$ |
|  | $0.5 \pm 0.1$ | 100 | $0.9 \pm 0.1$ (max) |
| Polylysine Mr = 21600 | $1.2 \pm 0.5$ | 100 | $0.13 \pm 0.05$ |
|  | $0.3 \pm 0.1$ | 100 | $0.4 \pm 0.03$ |
|  |  |  |  |
| Ca$^{2+}$ in PBS | 0.2 | 100 | $0.25 \pm 0.05$ (max) |

* heptapeptide (see Figure 1C)

** heptalysine (see Figure 1A)

*** nonapeptide (see Figure 1B)
max = maximal turbidity observed

Table 1   (continued)

| Substance | Charge ratio (Taurocholate/ precipitant) | Precipitation (%) | Turbidity (OD 500 nm) |
|---|---|---|---|
| In 50 mM Tris-HCl, pH=7.4 | 2 | 0 | $0.13 \pm 0.03$ |
| | 1 | 0 | $0.12 \pm 0.03$ |
| | 0.2 | 0 | $0.23 \pm 0.06$ (max) |
| $Mg^{2+}$ in PBS | 5 | 0 | 0 |
| | 0.2 | 0 | 0 |
| | | | |
| Lysine | 1 | 0 | 0 |
| | 0.25 | 0 | 0 |
| Arginine | 1 | 0 | 0 |
| | 0.25 | 0 | 0 |
| | | | |
| 1,6 Hexanediamine | 0.165 | 0 | 0 |
| | 0.1 | 0 | 0 |
| 1,10 Diaminodecane | 3 | 0 | 0 |
| | 1 | 0 | 0 |
| | 0.5 | 0 | 0 |
| 1,12 Diaminododecane | 2 | $13 \pm 4$ | $0.01 \pm 0.005$ |
| | 1 | $17 \pm 4$ | $0.01 \pm 0.005$ |
| | 0.5 | $65 \pm 5$ | $0.02 \pm 0.01$ |
| | 0.125 | 100 | $0.4 \pm 0.05$ (max) |
| | | | |
| Polylysine Mr 21600 in 10 mM Arg and 10 mM Lys | 0.6 | 100 | $1.3 \pm 0.1$ |
| | 0.3 | 100 | $1.6 \pm 0.2$ |

## Claims

1. Precipitant comprising or being able to generate a cation with at least two positive charges for the reduction of the uptake of cholesterol, esters of cholesterol and lipid from the gut.

2. Precipitant comprising or being able to generate a cation with at least two positive charges as agent for the treatment and prevention of hyperlipidaemia, including hypercholesterolaemia, the management of obesity, atherosclerosis, myocardial infarction and stroke.

3. The precipitant of claim 1 or 2, wherein the charges are thus distributed within the agent that they allow contact to at least two cholesterol and lipids comprising micelles or vesicles.

4. The precipitant of one of claims 1 to 3 that comprises at least two quaternary ammonium groups.

5. The precipitant of claim 4 that comprises at least two, preferably at least 10 lysines and/or arginins.

6. The precipitant of claim 5 that is polylysin with an average molecular weight of at least 2300.

7. The precipitant of claim 4 that is a protein or peptide, in particular a β-peptide.

8. The precipitant of one of claims 1 to 7, characterized in that the anion is a deprotonated strong acid, preferably a monoacid.

9. The precipitant of claim 8, characterized in that the anion is chloride.

10. β-peptide as agent for the treatment and/or prevention of hyperlipidaemia, including hypercholesterolaemia, the management of obesity, atheriosclerosis, myocardial infarction and stroke.

11. The β-peptide of claim 10 that comprises a helix with amphipathic character.

12. The β-peptide of claim 10 or 11 that comprises at least two positive charges.

13. A pharmaceutical composition comprising a precipitant or β-peptide of one of claims 1 to 12 together with a suitable carrier.

14. The composition of claim 13 that is an oral composition.

15. The composition of claim 13 or 14, characterized in that it disintegrates in the gut.

**Fig. 1A**

**Fig. 1B**

Fig. 1C

Fig. 1D

8

Fig. 2

Effect of Polylysine (Mr=2300) on Micelles

Fig. 3

**European Patent Office**

## PARTIAL EUROPEAN SEARCH REPORT

which under Rule 45 of the European Patent Convention
shall be considered, for the purposes of subsequent
proceedings, as the European search report

Application Number

EP 99 81 0892

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 97 47593 A (NOVARTIS AG ET AL) 18 December 1997 (1997-12-18) * claims 1-7 * * page 11, last paragraph - page 13, last paragraph * --- | 1-4,7, 10-15 | A61K31/14 A61K38/08 A61P3/04 A61P3/06 |
| X | EP 0 622 078 A (HISAMITSU PHARMACEUTICAL CO) 2 November 1994 (1994-11-02) * claim 1 * --- | 1-4, 13-15 | |
| X | DE 15 93 970 A (ROUSSEL-UCLAF) 17 February 1972 (1972-02-17) * claims 1-7 * * page 1, last paragraph - page 2, line 4 * --- | 1-5,8,9, 13-15 | |
| X | BE 671 478 A (ECHERCH ET INDUSTRIE THERAPEUTIQUES R.I.T) 27 April 1966 (1966-04-27) * claims 1-8 * --- -/-- | 1-4,8,9, 13-15 | TECHNICAL FIELDS SEARCHED (Int.Cl.7) A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| BERLIN | 23 March 2000 | Siatou, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**INCOMPLETE SEARCH
SHEET C**

Application Number

EP 99 81 0892

```
Claim(s) searched completely:
      5-12

Claim(s) searched incompletely:
      1-4, 13-15

Reason for the limitation of the search:

Present claims 1-4 and 13-15, as far as they refer to compositions
containing compounds as defined in claims 1-4,  relate to an extremely
large number of compounds and/or compositions defined by reference to a
desirable characteristic or property, namely reduction of cholesterol and
lipid uptake in the gut.
The claims cover all compounds and/or compositions having this
characteristic or property, whereas the application provides support
within the meaning of Article 84 EPC and/or disclosure within the meaning
of Article 83 EPC for only a very limited number of compounds. In the
present case, the claims so lack support, and the application so lacks
disclosure, that a meaningful search over the whole of the claimed scope
is impossible. Independent of the above reasoning, the claims also lack
clarity (Article 84 EPC). An attempt is made to define the compounds
and/or compositions by reference to a result to be achieved. Again, this
lack of clarity in the present case is such as to render a  meaningful
search over the whole of the claimed scope impossible.
Consequently, the search has been carried out for those parts of the
application which do appear to be clear (and concise), namely the
compounds described in claims 5-12, those mentioned in the description at
page 4, lines 11-24, page 9, and Fig. 1A-1D.
```

**European Patent Office**

**PARTIAL EUROPEAN SEARCH REPORT**

Application Number

EP 99 81 0892

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X,D | S. ABBELE ET AL: "(S)-beta-3-Homolysine- and (S)-beta-3-Homoserine-Containing beta-Peptides: CD Spectra in Aqueous Solution" HELVETICA CHIMICA ACTA, vol. 81, 1998, pages 2141-2156, XP002133823 * page 2142 * | 1-4,7, 10-12 | |
| X,D | D. SEEBACH ET AL: "Pleated Sheets and Turns of beta-Peptides with Proteinogenic Side Chains" ANGEW. CHEM. INT. ED., vol. 38, no. 11, 1999, pages 1595-1597, XP002133824 * page 1595, right-hand column * * page 1597, left-hand column * | 10,11 | TECHNICAL FIELDS SEARCHED (Int.Cl.7) |
| A,D | WO 97 36927 A (BOFFELLI DARIO ;HAUSER HELMUT (CH)) 9 October 1997 (1997-10-09) * claims 1-53 * | 1-15 | |
| A,D | H. HAUSER ET AL: "Identification of a receptor mediating absorption of dietary cholesterol in the intestine" BIOCHEMISTRY, vol. 37, 1998, pages 17843-17850, XP002133825 * abstract * | 1-15 | |

EPO FORM 1503 03.82 (P04C10)

13

**EP 1 090 634 A1**

ANNEX TO THE EUROPEAN SEARCH REPORT
ON EUROPEAN PATENT APPLICATION NO.

EP 99 81 0892

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2000

| Patent document cited in search report | | | Publication date | Patent family member(s) | | | Publication date |
|---|---|---|---|---|---|---|---|
| WO 9747593 | A | | 18-12-1997 | AU | 3258197 | A | 07-01-1998 |
| | | | | CA | 2256516 | A | 18-12-1997 |
| | | | | EP | 0906272 | A | 07-04-1999 |
| EP 0622078 | A | | 02-11-1994 | AT | 183924 | T | 15-09-1999 |
| | | | | AU | 3266793 | A | 03-08-1993 |
| | | | | CA | 2117371 | A | 22-07-1993 |
| | | | | DE | 69326231 | D | 07-10-1999 |
| | | | | DE | 69326231 | T | 30-12-1999 |
| | | | | EP | 0835888 | A | 15-04-1998 |
| | | | | GR | 3031583 | T | 31-01-2000 |
| | | | | WO | 9313781 | A | 22-07-1993 |
| | | | | JP | 2712056 | B | 10-02-1998 |
| | | | | KR | 179675 | B | 20-03-1999 |
| | | | | US | 5665348 | A | 09-09-1997 |
| | | | | US | 5800809 | A | 01-09-1998 |
| DE 1593970 | A | | 17-02-1972 | CH | 474480 | A | 30-06-1969 |
| | | | | DK | 122661 | B | 27-03-1972 |
| | | | | FR | 1584708 | A | 02-01-1970 |
| | | | | GB | 1188013 | A | 15-04-1970 |
| | | | | IL | 28166 | A | 24-03-1971 |
| | | | | NL | 6708455 | A | 29-12-1967 |
| | | | | FR | 6473 | M | 18-11-1968 |
| BE 671478 | A | | 27-04-1966 | NONE | | | |
| WO 9736927 | A | | 09-10-1997 | AU | 710061 | B | 09-09-1999 |
| | | | | AU | 2174197 | A | 22-10-1997 |
| | | | | CA | 2249459 | A | 09-10-1997 |
| | | | | CN | 1216995 | A | 19-05-1999 |
| | | | | EP | 0889906 | A | 13-01-1999 |
| | | | | NO | 984524 | A | 30-11-1998 |

EPO FORM P0459

For more details about this annex : see Official Journal of the European Patent Office, No. 12/82

14